# EUROPEAN PATENT SPECIFICATION

(11) **EP 2 815 763 B1**
(45) Date of publication and mention of the grant of the patent: **13.06.2018**
(21) Application number: 13751092.1
(22) Date of filing: 22.02.2013
(51) Int. Cl.: A61K 39/395, A61P 13/12, A61P 29/00, A61P 31/04, C07K 7/06, C07K 16/44, C12N 5/10, C12N 15/09, C07K 7/08, C07K 16/18, A61K 39/00

(54) **THERAPEUTIC AGENT FOR INFLAMMATORY DISEASE**
HEILMITTEL FÜR ENTZÜNDUNGSERKRANKUNGEN
AGENT THÉRAPEUTIQUE POUR MALADIE INFLAMMATOIRE

(30) Priority: 22.02.2012 JP 2012035965
(43) Date of publication of application: 24.12.2014
(73) Proprietor: Josai University Corporation, Tokyo 104-0092 (JP)
(72) Inventor: SATO, Shuji, Kisarazu-shi Chiba-ken 292-0818 (JP); GOTO, Takeshi, Kisarazu-shi Chiba-ken 292-0818 (JP); OHMORI, Naoya, Kisarazu-shi Chiba-ken 292-0818 (JP); CHIANG, Kueichen, Kisarazu-shi Chiba-ken 292-0818 (JP); SHIMADA, Yayoi, Kisarazu-shi Chiba-ken 292-0818 (JP); INOMATA, Masafumi, Yufu-shi Oita 879-5593 (JP); KUSANO, Toru, Yufu-shi Oita 879-5593 (JP); HIRATSUKA, Takahiro, Yufu-shi Oita 879-5593 (JP); NOGUCHI, Takayuki, Yufu-shi Oita 879-5593 (JP); HAGIWARA, Satoshi, Yufu-shi Oita 879-5593 (JP)
(74) Representative: Prüfer & Partner mbB Patentanwälte · Rechtsanwälte
(86) International application number: PCT/JP2013/054551
(87) International publication number: WO 2013/125687

(56) References cited:
- EP-A1- 1 820 855
- EP-A1- 2 196 212
- WO-A1-2006/025580
- WO-A1-2012/023614
- WO-A1-2012/023614
- JP-A- 2011 503 012
- US-A1- 2009 117 099
- JUN XU ET AL: "Extracellular histones are major mediators of death in sepsis", NATURE MEDICINE, vol. 15, no. 11, 1 November 2009 (2009-11-01), pages 1318-1321, XP055082137, ISSN: 1078-8956, DOI: 10.1038/nm.2053 & CATHERINE CHAPUT ET AL: "Sepsis: the dark side of histones", NATURE MEDICINE, vol. 15, no. 11, 1 November 2009 (2009-11-01), pages 1245-1246, XP055194652, ISSN: 1078-8956, DOI: 10.1038/nm1109-1245
- HAI HUANG ET AL: "Endogenous histones function as alarmins in sterile inflammatory liver injury through Toll-like receptor 9 in mice", HEPATOLOGY, vol. 54, no. 3, 2 September 2011 (2011-09-02), pages 999-1008, XP055194650, ISSN: 0270-9139, DOI: 10.1002/hep.24501
- K.-C. CHIANG ET AL: "A Novel Peptide Mimotope Identified As a Potential Immunosuppressive Vaccine for Organ Transplantation", THE JOURNAL OF IMMUNOLOGY, vol. 182, no. 7, 1 April 2009 (2009-04-01) , pages 4282-4288, XP055082127, ISSN: 0022-1767, DOI: 10.4049/jimmunol.0800641
- T. A. FUCHS ET AL: "Histones induce rapid and profound thrombocytopenia in mice", BLOOD, vol. 118, no. 13, 23 June 2011 (2011-06-23), pages 3708-3714, XP055194648, ISSN: 0006-4971, DOI: 10.1182/blood-2011-01-332676
- TORU KUSANO ET AL: "A Novel Anti-Histone H1 Monoclonal Antibody, SSV Monoclonal Antibody, Improves Lung Injury and Survival in a Mouse Model of Lipopolysaccharide-Induced Sepsis-Like Syndrome", BIOMED RESEARCH INTERNATIONAL, vol. 61, no. 4, 1 January 2015 (2015-01-01), pages 1496-10, XP055194646, ISSN: 2314-6133, DOI: 10.1002/eji.1830260726
- KUEI-CHEN CHIANG, ET AL.: 'A Novel Peptide Mimotope Identified As a Potential Immunosuppressive Vaccine for Organ Transplantation' J.IMMUNOL. vol. 182, 01 April 2009, pages 4282 - 4288, XP055082127
- JUN XU, ET AL.: 'Extracellular histones are major mediators of death in sepsis' NATURE MEDICINE vol. 15, no. 11, 2009, pages 1318 - 1322, XP055082137
- JUN XU, ET AL.: 'Extracellular Histones Are Mediators of Death through TLR2 and TLR4 in Mouse Fatal Liver Injury' J.IMMUNOL. vol. 187, 2011, pages 2626 - 2631, XP055082161

### Sequence Listing

### SEQUENCE LISTING

<110> Josai university corporation
<120> Therapeutic agent for inflammatory disease
<130> 198711PX
<150> JP 2012-035965
   <151> 2012-02-22
<160> 13
<170> PatentIn version 3.4
<210> 1
   <211> 12
   <212> PRT
   <213> Artificial
<220>
   <223> SSV PEPTIDE
<400> 1
   Ser Ser Val Leu Tyr Gly Gly Pro Pro Ser Ala Ala
   1 5 10
<210> 2
   <211> 10
   <212> PRT
   <213> Artificial
<220>
   <223> SSV Mab- LIGHT CHAIN VARIABLE REGION CDR1
<400> 2
   Arg Ala Ser Ser Ser Val Ser Tyr Met His
   1 5 10
<210> 3
   <211> 7
   <212> PRT
   <213> Artificial
<220>
   <223> SSV Mab- LIGHT CHAIN VARIABLE REGION CDR2
<400> 3
   Ala Thr Ser Asn Leu Ala Ser
   1 5
<210> 4
   <211> 9
   <212> PRT
   <213> Artificial
<220>
   <223> SSV Mab- LIGHT CHAIN VARIABLE REGION CDR3
<400> 4
   Gln Gln Trp Ser Ser Asn Pro Trp Thr
   1 5
<210> 5
   <211> 384
   <212> DNA
   <213> Artificial
<220>
   <223> SSV Mab- LIGHT CHAIN VARIABLE REGION DNA
<400> 5
<210> 6
   <211> 128
   <212> PRT
   <213> Artificial
<220>
   <223> SSV Mab- LIGHT CHAIN VARIABLE REGION PRT
<400> 6
   Met Asp Phe Gln Val Gln Ile Phe Ser Phe Leu Leu Ile Ser Ala Ser
   1 5 10 15
   Val Ile Met Ser Arg Gly Gln Ile Val Leu Ser Gln Ser Pro Ala Ile
   20 25 30
   Leu Ser Ala Ser Pro Gly Glu Lys Val Thr Met Thr Cys Arg Ala Ser
   35 40 45
   Ser Ser Val Ser Tyr Met His Trp Tyr Gln Gln Lys Pro Gly Ser Ser
   50 55 60
   Pro Lys Pro Trp Ile Tyr Ala Thr Ser Asn Leu Ala Ser Gly Val Pro
   65 70 75 80
   Ala Arg Phe Ser Gly Ser Gly Ser Gly Thr Ser Tyr Ser Leu Thr Ile
   85 90 95
   Ser Arg Val Glu Ala Glu Asp Ala Ala Thr Tyr Tyr Cys Gln Gln Trp
   100 105 110
   Ser Ser Asn Pro Trp Thr Phe Gly Gly Gly Thr Lys Leu Glu Ile Lys
   115 120 125
<210> 7
   <211> 5
   <212> PRT
   <213> Artificial
<220>
   <223> SSV Mab- HEAVY CHAIN VARIABLE REGION CDR1
<400> 7
   Gly Tyr Asn Met Asn
   1 5
<210> 8
   <211> 17
   <212> PRT
   <213> Artificial
<220>
   <223> SSV Mab- HEAVY CHAIN VARIABLE REGION CDR2
<400> 8
   Asn Ile Asn Pro Tyr Tyr Gly Ser Thr Ser Tyr Asn Gln Lys Phe Lys
   1 5 10 15
   Gly
<210> 9
   <211> 13
   <212> PRT
   <213> Artificial
<220>
   <223> SSV Mab- HEAVY CHAIN VARIABLE REGION CDR3
<400> 9
   Ser Pro Tyr Tyr Ser Asn Tyr Trp Arg Tyr Phe Asp Tyr
   1 5 10
<210> 10
   <211> 423
   <212> DNA
   <213> Artificial
<220>
   <223> SSV Mab- HEAVY CHAIN VARIABLE REGION DNA
<400> 10
   atgggatgga gctggatctt tctcttcctt ctgtcagtaa ctgcaggtgt ccactctgag 60
   atccagctgc agcagtctgg agctgagctg gtgaagcctg gggcttcagt gaagatatcc 120
   tgcaaggctt ctggttactc attcactggc tacaacatga actgggtgaa gcagagccat 180
   ggaaagagcc ttgagtggat tggaaatatt aatccttact atggtagtac tagctacaat 240
   cagaagttca agggcaaggc cacattgact gtagacaaat cttccagcac agcctacatg 300
   cagctcaaca gcctgacatc tgaggactct gcagtctatt actgtgcaag aagtccctac 360
   tatagtaact actggaggta ctttgactac tggggccaag gcaccactct cacagtctcc 420
   tca 423
<210> 11
   <211> 141
   <212> PRT
   <213> Artificial
<220>
   <223> SSV Mab- HEAVY CHAIN VARIABLE REGION PRT
<400> 11
   Met Gly Trp Ser Trp Ile Phe Leu Phe Leu Leu Ser Val Thr Ala Gly
   1 5 10 15
   Val His Ser Glu Ile Gln Leu Gln Gln Ser Gly Ala Glu Leu Val Lys
   20 25 30
   Pro Gly Ala Ser Val Lys Ile Ser Cys Lys Ala Ser Gly Tyr Ser Phe
   35 40 45
   Thr Gly Tyr Asn Met Asn Trp Val Lys Gln Ser His Gly Lys Ser Leu
   50 55 60
   Glu Trp Ile Gly Asn Ile Asn Pro Tyr Tyr Gly Ser Thr Ser Tyr Asn
   65 70 75 80
   Gln Lys Phe Lys Gly Lys Ala Thr Leu Thr Val Asp Lys Ser Ser Ser
   85 90 95
   Thr Ala Tyr Met Gln Leu Asn Ser Leu Thr Ser Glu Asp Ser Ala Val
   100 105 110
   Tyr Tyr Cys Ala Arg Ser Pro Tyr Tyr Ser Asn Tyr Trp Arg Tyr Phe
   115 120 125
   Asp Tyr Trp Gly Gln Gly Thr Thr Leu Thr Val Ser Ser
   130 135 140
<210> 12
   <211> 26
   <212> DNA
   <213> Artificial
<220>
   <223> PRIMER
<400> 12
   caccatggag ttagtttggg cagcag 26
<210> 13
   <211> 23
   <212> DNA
   <213> Artificial
<220>
   <223> PRIMER
<400> 13
   cacgactgag gcacctccag atg 23

### SEQUENCE LISTING

<110> Josai university corporation
<120> Therapeutic agent for inflammatory disease
<130> 198711PX
<150> JP 2012-035965
   <151> 2012-02-22
<160> 13
<170> Patentln version 3.4
<210> 1
   <211> 12
   <212> PRT
   <213> Artificial
<220>
   <223> SSV PEPTIDE
<400> 1
   Ser Ser Val Leu Tyr Gly Gly Pro Pro Ser Ala Ala
   1 5 10
<210> 2
   <211> 10
   <212> PRT
   <213> Artificial
<220>
   <223> SSV Mab- LIGHT CHAIN VARIABLE REGION CDR1
<400> 2
   Arg Ala Ser Ser Ser Val Ser Tyr Met His
   1 5 10
<210> 3
   <211> 7
   <212> PRT
   <213> Artificial
<220>
   <223> SSV Mab- LIGHT CHAIN VARIABLE REGION CDR2
<400> 3
   Ala Thr Ser Asn Leu Ala Ser
   1 5
<210> 4
   <211> 9
   <212> PRT
   <213> Artificial
<220>
   <223> SSV Mab- LIGHT CHAIN VARIABLE REGION CDR3
<400> 4
   Gln Gln Trp Ser Ser Asn Pro Trp Thr
   1 5
<210> 5
   <211> 384
   <212> DNA
   <213> Artificial
<220>
   <223> SSV Mab- LIGHT CHAIN VARIABLE REGION DNA
<400> 5
<210> 6
   <211> 128
   <212> PRT
   <213> Artificial
<220>
   <223> SSV Mab- LIGHT CHAIN VARIABLE REGION PRT
<400> 6
   Met Asp Phe Gln Val Gln Ile Phe Ser Phe Leu Leu Ile Ser Ala Ser
   1               5                   10                  15
   Val Ile Met Ser Arg Gly Gln Ile Val Leu Ser Gln Ser Pro Ala Ile
               20                  25                  30
   Leu Ser Ala Ser Pro Gly Glu Lys Val Thr Met Thr Cys Arg Ala Ser
           35                  40                  45
   Ser Ser Val Ser Tyr Met His Trp Tyr Gln Gln Lys Pro Gly Ser Ser
       50                  55                  60
   Pro Lys Pro Trp Ile Tyr Ala Thr Ser Asn Leu Ala Ser Gly Val Pro
   65                  70                  75                  80
   Ala Arg Phe Ser Gly Ser Gly Ser Gly Thr Ser Tyr Ser Leu Thr Ile
                   85                  90                  95
   Ser Arg Val Glu Ala Glu Asp Ala Ala Thr Tyr Tyr Cys Gln Gln Trp
               100                 105                 110
   Ser Ser Asn Pro Trp Thr Phe Gly Gly Gly Thr Lys Leu Glu Ile Lys
           115                 120                 125
<210> 7
   <211> 5
   <212> PRT
   <213> Artificial
<220>
   <223> SSV Mab- HEAVY CHAIN VARIABLE REGION CDR1
<400> 7
   Gly Tyr Asn Met Asn
   1               5
<210> 8
   <211> 17
   <212> PRT
   <213> Artificial
<220>
   <223> SSV Mab- HEAVY CHAIN VARIABLE REGION CDR2
<400> 8
   Asn Ile Asn Pro Tyr Tyr Gly Ser Thr Ser Tyr Asn Gln Lys Phe Lys
   1 5 10 15
   Gly
<210> 9
   <211> 13
   <212> PRT
   <213> Artificial
<220>
   <223> SSV Mab- HEAVY CHAIN VARIABLE REGION CDR3
<400> 9
   Ser Pro Tyr Tyr Ser Asn Tyr Trp Arg Tyr Phe Asp Tyr
   1 5 10
<210> 10
   <211> 423
   <212> DNA
   <213> Artificial
<220>
   <223> SSV Mab- HEAVY CHAIN VARIABLE REGION DNA
<400> 10
   atgggatgga gctggatctt tctcttcctt ctgtcagtaa ctgcaggtgt ccactctgag 60
   atccagctgc agcagtctgg agctgagctg gtgaagcctg gggcttcagt gaagatatcc 120
   tgcaaggctt ctggttactc attcactggc tacaacatga actgggtgaa gcagagccat 180
   ggaaagagcc ttgagtggat tggaaatatt aatccttact atggtagtac tagctacaat 240
   cagaagttca agggcaaggc cacattgact gtagacaaat cttccagcac agcctacatg 300
   cagctcaaca gcctgacatc tgaggactct gcagtctatt actgtgcaag aagtccctac 360
   tatagtaact actggaggta ctttgactac tggggccaag gcaccactct cacagtctcc 420
   tca 423
<210> 11
   <211> 141
   <212> PRT
   <213> Artificial
<220>
   <223> SSV Mab- HEAVY CHAIN VARIABLE REGION PRT
<400> 11
   Met Gly Trp Ser Trp Ile Phe Leu Phe Leu Leu Ser Val Thr Ala Gly
   1               5                   10                  15
   Val His Ser Glu Ile Gln Leu Gln Gln Ser Gly Ala Glu Leu Val Lys
               20                  25                  30
   Pro Gly Ala Ser Val Lys Ile Ser Cys Lys Ala Ser Gly Tyr Ser Phe
           35                  40                  45
   Thr Gly Tyr Asn Met Asn Trp Val Lys Gln Ser His Gly Lys Ser Leu
       50                  55                  60
   Glu Trp Ile Gly Asn Ile Asn Pro Tyr Tyr Gly Ser Thr Ser Tyr Asn
   65                  70                  75                  80
   Gln Lys Phe Lys Gly Lys Ala Thr Leu Thr Val Asp Lys Ser Ser Ser
                   85                  90                  95
   Thr Ala Tyr Met Gln Leu Asn Ser Leu Thr Ser Glu Asp Ser Ala Val
               100                 105                 110
   Tyr Tyr Cys Ala Arg Ser Pro Tyr Tyr Ser Asn Tyr Trp Arg Tyr Phe
           115                 120                 125
   Asp Tyr Trp Gly Gln Gly Thr Thr Leu Thr Val Ser Ser
       130                 135                 140
<210> 12
   <211> 26
   <212> DNA
   <213> Artificial
<220>
   <223> PRIMER
<400> 12
   caccatggag ttagtttggg cagcag 26
<210> 13
   <211> 23
   <212> DNA
   <213> Artificial
<220>
   <223> PRIMER
<400> 13
   cacgactgag gcacctccag atg 23

## Description

### Cross Reference to Related Applications

The present application claims priority to Japan Patent Application No. 2012-35965 (filed on February 22, 2012), which is hereby incorporated by reference in its entirety.

### Technical Field

The present invention relates to a novel therapeutic agent for inflammatory disease comprising as an effective ingredient a monoclonal antibody or an antigen binding fragment thereof.

### Background Art

In recent years, a histone which is an intranuclear component has been reported to be Damage-associated molecular pattern molecules (DAMPs). Such DAMPs are drawing attention as a cause of inflammatory diseases such as organ injury.

On the other hand, sepsis is a severe systemic infectious disease in which bacteria continuously or intermittently enter the blood from an infection focus, which is caused by diseases such as infectious diseases, cirrhosis, renal failure, diabetes and dystocia, or by therapies against injury or disease, such as indwelling catheter, transfusion device, dialysis or tracheostomy. In its broader sense, sepsis is not restricted to the invasion by a microorganism into a host, but is defined to include clinical conditions of infectious diseases, in which two or more of the following are met: (1) body temperature >38°C or <36°C; (2) heart rate >90 beats/min.; (3) frequency of respiration >20 breaths/min. or PaCO₂ < 32 mmHg; (4) number of leukocytes > 12,000/µl or <4000/µl, or ratio of stab neutrophil > 10%. Recently, the pathological conditions exhibiting these symptoms are called systemic inflammatory response syndrome (SIRS) (Non-patent Literature 1: Crit. Care Med., 20:864-874, 1992). Sepsis further includes organ dysfunction, severe sepsis complicated with hypoperfusion or hypotension, lactic acidosis, hypouresis and septic shock complicated with consciousness disorder (Non-patent Literature 2: Chest,101:1644-1655,1992). Severe sepsis and septic shock induce disseminated intravascular coagulation syndrome (DIC), adult respiratory distress syndrome (ARDS) and multiple organ dysfunction (MODS).

The causative bacteria of sepsis are mainly staphylococci, streptococci, Escherichia coli, Pseudomonas aeruginosa, Klebsiella and Enterobacter. By the infection of these bacteria, high fever, chill, tachycardia and strong systemic symptoms are exhibited, and existence of the infective bacteria is often confirmed in the arterial blood, venous blood, spinal fluid and bone marrow fluid.

Recently, due to the development of various strong antibiotics, sepsis caused by these bacteria is decreasing. However, sepsis caused by new bacteria such as MRSA, which acquired a resistant gene is increasing. Reflecting the increase of treatments using indwelling catheter or transfusion device, dialysis, and invasive treatments and surgery such as tracheostomy, there is a tendency that the larger the scale of the hospital, the more the occurrence of sepsis. Further, frequency of sepsis is increasing among those having poor resistance to infections, such as newborns, elder people, patients suffering from hematopoietic organ tumors and patients whose immunities are decreased due to administration of adenocorticotropic hormones or anticancer agents. Thus, sepsis is continuously increasing in spite of the development of medicine.

A method for prevention or therapy of sepsis now employed is carried out by administering the best antibiotic against the causative bacterium after detecting the causative bacterium and determining the sensitivities thereof to antibiotics, and by simultaneously promoting the defending ability of the host by fluid replacement, replenishment of electrolytes, improvement of hypoproteinemia, replenishment of nutrients, administration of γ-globulin and the like. In cases where the shock unfortunately appears, treatments such as removal of lesion by surgery, improvement of circulatory dysfunction, administration of opsonin-activating substances, administration of adenocorticotropic hormones, administration of synthetic protease inhibitors, and the like are carried out. However, since the symptoms of the underlying basal disease and the symptoms of sepsis overlap, clear diagnosis is not easily carried out, which often gives difficulty in the prevention and therapy of sepsis. In cases where the septic shock occurs, the prevention and therapy are difficult. Thus, sepsis is a disease which gives a high death rate even at present.

The death rate of sepsis varies from 10%-20% to 50% depending on the report. Forty percent of sepsis cases are complicated with septic shock, and the prognosis of the shock is bad. There is a report which shows the death rate of the shock is 77 to 90%. Therefore, the primary object of the therapy is the prevention of the septic shock. If the changes which occurs in the initial stage of the shock are grasped and early diagnosis is attained, early treatment can be attained and improvement of prognosis is expected. However, although a number of anti-shock drugs and therapeutic methods have been studied, almost none of them were judged effective.

It is thought that sepsis is caused by inflammatory cytokines such as tumor necrosis factor (TNF), interleukin 1 (IL-1), interleukin 6 (IL-6) and interleukin 8 (IL-8), which are excessively produced by monocytes, macrophages, vascular endothelial cells and the like in response to the infectious stimuli (such as bacterial cells per se, endotoxins, cell wall components which are peptide glycan/teichoic acid complexes and exotoxins). By the excessively produced inflammatory cytokines, eicosanoid and lipid mediators of platelet-activating factor are released, and the cytokine net work is activated by the interaction thereof, so that the inflammatory reaction is amplified. During this process, complement system, coagulation system, kinin system and adrenocorticotropic hormone/endorphin system are also activated, and the systemic inflammatory reaction of which underlying symptom is vascular endothelial disorder is induced. For expression of circulatory disorders or histotoxic disorders, participation of elastase originated from granulocytes and active oxygen has been shown.

Therefore, a number of clinical tests of the therapeutic methods which inhibit the inflammatory cytokines, represented by administration of substances that inhibit the inflammatory cytokines have been carried out. However, all of them were unsuccessful (Non-patent Literature 3: Lancet,351:929-933,1998, JAMA,271:1836-1843,1994).

Although those various therapeutic method have been studied, the death rate of sepsis is kept high and there are substantially no effective remedies. The reason therefor is that the pathological conditions of sepsis have not yet been completely understood (Non-patent Literature 4: Nath. J. Med.,55:132-141,1999).
Recently, it was shown in US 2009/117099 A1 that extracellular histones released in response to inflammatory challenge might be mediators, which contribute to endothelial dysfunction, organ failure and death during sepsis. As such, the authors concluded that they can be targeted pharmacologically by inhibitors, for instance anti-histone blocking antibodies, as well as used as biomarkers for prognosis of sepsis and other diseases.
Similarly, Xu et al. showed that extracellular histones, mainly H3 and H4, appear to be both biomarkers of disease progression and therapeutic targets in sepsis and other inflammatory diseases (Non-patent Literature 5: Extracellular histones are major mediators of death in sepsis; Nat Med. 2009 November ; 15(11): 1318-1321).

Further, as another inflammatory disease in which DAMPs are involved, ischemia reperfusion injury has been reported. Still further, it has been reported that acute renal failure often occurs due to renal ischemia reperfusion injury. However, there are substantially no effective therapeutic method for such a diseased state.

Moreover, EP 1 820 855 A1 relates to anti-histone H1 antibodies, hybridomas for the production thereof, and polypeptides, which are useful for suppressing, predicting, or diagnosing transplant rejection in organ transplantation. For example EP 1 820 855 A1 discloses the hybridoma 16G9 producing an anti-histone H1 antibody that was also used in the context of noninvasively administering vaccine through the skin in EP 2 196 212 A1.

Therefore, creating an excellent therapeutic agent for inflammatory diseases in which DAMPs are involved is still needed.

### Citation List

### Non-Patent Literature

Non-Patent Literature 1: Crit. Care Med.,20:864-874,1992
Non-Patent Literature 2: Chest, 101:1644-1655,1992
Non-Patent Literature 3: Lancet, 351:929-933,1998, JAMA, 271:1836-1843, 1994
Non-Patent Literature 4: Nath. J. Med., 55:132-141, 1999
Non-patent Literature 5: Nat Med. 2009 November ; 15(11): 1318-1321)

### Summary of Invention

The present inventors have found that a monoclonal antibody or an antigen binding fragment thereof which can specifically recognize a peptide consisting of an amino acid sequence represented by SSVLYGGPPSAA (SEQ ID NO:1) has excellent advantageous effects on inflammatory diseases in which histone is involved. The present invention is based on these findings.

Therefore, an object of the present invention is to provide a novel therapeutic agent for inflammatory diseases.

Accordingly, the present invention provides a therapeutic agent for use in a treatment of an inflammatory disease as defined in claim 1. Accordingly, the therapeutic agent comprises a monoclonal antibody or an antigen binding fragment thereof which binds to a peptide consisting of an amino acid sequence represented by SSVLYGGPPSAA (SEQ ID NO:1) or a conjugate of the peptide and a pharmaceutically acceptable carrier.
Preferred embodiments are set forth in subclaims.

By the monoclonal antibody of the present invention or an antigen binding fragment thereof, inflammatory diseases can be treated effectively. Brief Description of Drawings

Fig. 1 shows the results from the tests for identifying the isotype of the monoclonal antibody of the present invention (hereinafter, also referred to as "SSVmAb").
Fig. 2 shows the results from the tests in which the binding affinities of the monoclonal antibody of the present invention (SSVmAb) for histone H1, histone H2A, H2B, H3 or H4 were compared.
Fig. 3 shows the results from the tests in which the binding affinities of the monoclonal antibody produced by hybridoma 16G9 (hereinafter, also referred to as "16G9mAb") for histone H1, histone H2A, H2B, H3 or H4 were compared. The hybridoma has been deposited under the deposition number FERM BP-10413 (Reference).
Fig. 4 shows the results from the tests for mixed lymphocyte reaction (MLR) using the monoclonal antibody of the present invention (SSVmAb) and 16G9mAb.
Fig. 5 shows the results from the comparison in which the reactivities of the monoclonal antibody of the present invention (SSVmAb) and 16G9mAb with T cells were compared by flow cytometry.
Fig. 6A shows the results from the MLR tests for the monoclonal antibody of the present invention (SSVmAb) and a control reagent (Isotype IgG1) using spleen cells in which ATP synthase is not knocked down by siRNA. Fig. 6B shows the results of the MLR tests for the monoclonal antibody of the present invention (SSVmAb) and a control reagent (Isotype IgG1) using spleen cells in which ATP synthase is knocked down by siRNA.
Fig. 7 shows the monoclonal antibody of the present invention (SSVmAb) increased the survival rate of septicemia model animals in Test Example 7.
Fig. 8 shows the monoclonal antibody of the present invention (SSVmAb) increased the survival rate of septicemia model animals in Test Example 8.
Fig. 9 shows the results of the measurements of the histone H1 concentration in blood sample (serum) and lung of control group and SSV mAb-administered group in Test Example 8. Fig. 9A is a graph showing the histone H1 concentration in blood sample. Fig. 9B is a graph showing the histone H1 concentration in lung.
Fig. 10 shows the results of the measurements of the histone H3 concentration in blood sample and lung of control group and SSV mAb-administered group in Test Example 8. Fig. 10A is a graph showing the histone H3 concentration in blood sample. Fig. 10B is a graph showing the histone H3 concentration in lung.
Fig. 11 shows the results of the measurements of the histone H4 concentration in blood sample and lung of control group and SSV mAb-administered group in Test Example 8. Fig. 11A is a graph showing the histone H4 concentration in blood sample. Fig. 11B is a graph showing the histone H4 concentration in lung.
Fig. 12 shows micrographs of lung tissue sections which were obtained from healthy rat, and from rats of SSV mAb-administered group and control group after the test termination of Test Example 8 and then stained. Figl2A shows a micrograph from a healthy rat. Fig12B shows a micrograph from a SSV mAb-administered group. Fig12C shows a micrograph from a control group.
Fig. 13 shows the results of evaluating congestion, edema, inflammation and bleeding in rats of SSV mAb-administered group and control group after the test termination of Test Example 8.
Fig. 14 shows the results of measuring the concentrations in blood samples of inflammatory cytokine (TNF-α, IL-1β, IL-6) and inhibitory cytokine (IL-10) of control group and SSV mAb-administered group in Test Example 8. Fig. 14A shows the TNF-α concentration in blood sample. Fig. 14B shows the IL-1β concentration in blood sample. Fig. 14C shows the IL-6 concentration in blood sample. Fig. 14D shows the IL-10 concentration in blood sample.

### Detailed Description of the Invention

### Deposition

The hybridoma of the present invention Mouse-Mouse hybridoma SSV-C 93-3 was deposited at National Institute of Technology and Evaluation, Patent Microorganisms Depositary (Address: Biotechnology Headquarter, 2-5-8 Kazusa Kamatari, Kisarazu-shi, Chiba-ken, Japan) on the original deposition day of August 17, 2010 under the deposition number NITE BP-972.

### Monoclonal antibody and hybridoma

One characteristics of the monoclonal antibody of the present invention or an antigen binding fragment thereof is that the monoclonal antibody of the present invention or an antigen binding fragment thereof binds to a peptide consisting of an amino acid sequence represented by SSVLYGGPPSAA (SEQ ID NO:1) or a conjugate of the peptide and a pharmaceutically acceptable carrier. Surprisingly, the present inventors have found that such a monoclonal antibody or an antigen binding fragment thereof has an excellent therapeutic effect on inflammatory diseases.

Inflammatory diseases in the present invention are preferably inflammatory diseases in which histone is involved, more preferably acute inflammatory diseases, more preferably sepsis, renal ischemia reperfusion injury or renal failure, still more preferably sepsis, renal ischemia reperfusion injury or acute renal failure, still further preferably sepsis.

According to the present invention, the above-mentioned antibody or an antigen binding fragment thereof is against a peptide consisting of an amino acid sequence represented by SSVLYGGPPSAA (SEQ ID NO:1) or, the peptide and a pharmaceutically acceptable carrier.

Further, according to the present invention, the above-mentioned antibody or an antigen binding fragment thereof specifically binds to histone H1, histone H3 and histone H4. An antibody or an antigen binding fragment thereof which has such binding capacity may be especially advantageously used in the treatment of inflammatory diseases as shown in 8-2 in Example hereinbelow described.

Further, it is possible according to the present invention that the above-mentioned antibody or an antigen binding fragment thereof has a higher binding affinity for core histone than for linker histone (histone H1).

According to another preferred aspect of the present invention, core histone is histone H2A, H2B, H3 or H4, and more preferably H2A, H3 or H4.

The antibody of the present invention or an antibody binding fragment thereof also comprises a heavy chain and/or a light chain. Each of a light chain and a heavy chain has a variable region at its N-terminal, and each variable region contains four framework regions (FR) and three complementarity determining regions (CDR) in an alternate fashion. Conventionally, residues in a variable region are numbered according to the system devised by Kabat et al. The system is described in Kabat et al., 1987, Sequences of Proteins of Immunological Interest, US Department of Health and Human Services, NIH, USA. Unless otherwise stated, this numbering system is used in the present specification. Numbering based on the method by Kabat et al. can be easily performed, for example, using the web site at http://www.bioinf.org.uk/abysis/tools/analyze.cgi.

The Kabat nomenclature of residues does not necessarily correspond to the linear numbering of amino acid residues directly. An actual linear amino acid sequence in either a structural element of the basic structure of a variable region, a framework or a CDR may have a fewer or additional amino acid compared with the strict Kabat numbering depending on its truncation or insertion. For a given antibody, correct Kabat numbering of residues will be determined by aligning homologous residues in a sequence numbered according to the "standard" Kabat numbering and in a sequence of the antibody.

The light chain variable region of the antibody of the present invention or an antigen binding fragment thereof comprises CDR1 consisting of an amino acid sequence represented by RASSSVSYMH (SEQ ID NO:2), CDR2 consisting of an amino acid sequence represented by ATSNLAS (SEQ ID NO:3) and CDR3 consisting of an amino acid sequence represented by QQWSSNPWT (SEQ ID NO:4). The above-mentioned light chain variable region comprises an amino acid sequence represented by Position 23 to Position 128 of SEQ ID NO:6.

The heavy chain variable region of the antibody of the present invention or an antigen binding fragment thereof comprises CDR1 consisting of an amino acid sequence represented by GYNMN (SEQ ID NO:7), CDR2 consisting of an amino acid sequence represented by NINPYYGSTSYNQKFKG (SEQ ID NO:8) and CDR3 consisting of an amino acid sequence represented by SPYYSNYWRYFDY (SEQ ID NO:9). The above-mentioned heavy chain variable region comprises an amino acid sequence represented by Position 20 to Position 141 of SEQ ID NO:11.

Further, according to the present invention, the antibody of the present invention or an antigen binding fragment thereof comprises a light chain variable region comprising CDR1 consisting of an amino acid sequence represented by RASSSVSYMH (SEQ ID NO:2), CDR2 consisting of an amino acid sequence represented by ATSNLAS (SEQ ID NO:3) and CDR3 consisting of an amino acid sequence represented by QQWSSNPWT (SEQ ID NO:4), and a heavy chain variable region comprising a heavy chain variable region comprising CDR1 consisting of an amino acid sequence represented by GYNMN (SEQ ID NO:7), CDR2 consisting of an amino acid sequence represented by NINPYYGSTSYNQKFKG (SEQ ID NO:8) and CDR3 consisting of an amino acid sequence represented by SPYYSNYWRYFDY (SEQ ID NO:9).

Furthermore, the antibody of the present invention or antigen binding fragment thereof comprises a light chain variable region comprising an amino acid sequence represented by Position 23 to Position 128 of SEQ ID NO:6 and a heavy chain variable region comprising an amino acid sequence represented by Position 20 to Position 141 of SEQ ID NO:11.

Moreover, according to a preferred aspect of the present invention, the above-mentioned monoclonal antibody or an antigen binding fragment thereof can down-regulate the activity of ATP synthase. In addition, according to a more preferred aspect of the present invention, the above-mentioned ATP synthase is mitochondria ATP synthase.

The above-mentioned binding affinity and the down-regulation activity of ATP synthase activity of the monoclonal antibody of the present invention or an antigen binding fragment thereof are determined, for example, by the methods described in Test Examples 2 and 4 of the present specification.

Further, the monoclonal antibody of the present invention is preferably a chimeric antibody. Those skilled in the art can produce these antibodies according to known technologies in the art as described in, for example, Morrison, S.L., Oi, V. T., "immunoglobulin genes" Academic Press (London), 260-274 (1989); Roguska, M. L. et. Al., Humanization of murine monoclonal antibodies through variable domain resurfacing, Proc. Natl. Acad. Sci. USA, 91, 969-973 (1994); Tomizuka, K. et. al. Functional expression and germline transmission of a human chromosome fragment in chimaeric mice, Nature Genet., 16, 133-143 (1997); Winter, G. et. al., Making antibodies by phage display technology, Ann. Rev. Immunol., 12, 433-455 (1994); Griffiths, A. D. et. al., Isolation of high affinity human antibodies directly from large synthetic repertoires, EMBO. J., 13, 3245-3260 (1994).

Furthermore, according to a preferred aspect of the present invention, the above-mentioned antigen binding fragment is preferably Fab, Fab', (Fab')₂, Fv or scFv.

In addition, according to another preferred aspect of the present invention, the above-mentioned monoclonal antibody or an antigen binding fragment thereof is produced by a hybridoma Mouse-Mouse hybridoma SSV-C93-3.

The monoclonal antibody of the present invention or an antigen binding fragment thereof, and a hybridoma can be produced, for example, as follows. That is, first, the hybridoma of the present invention can be obtained using a peptide comprising an amino acid sequence represented by SSVLYGGPPSAA (SEQ ID NO:1) or a conjugate of this peptide and a pharmaceutically acceptable carrier as an antigen by fusing mammalian plasma cells (immune cells) immunized by this sensitizing antigen with mammalian myeloma cells, and cloning and screening the resulting hybridomas. Then the monoclonal antibody of the present invention can be obtained by culturing the hybridoma of the present invention and collecting antibody produced by it.

For methods of immunizing a mammal, any common administration methods in the art can be used. In particular, they include intraperitoneal injection, intrasplenic injection, intramuscular injection, subcutaneous injection, intradermal injection, oral administration, transmucosal administration, transdermal administration, but preferably they are intraperitoneal injection, intrasplenic injection. The dosage interval of a sensitizing antigen is appropriately determined depending on a dose of the sensitizing antigen, a species of the mammal and the like. For example, it can be several times per month.

Mammals to be immunized are not particularly limited, but preferably selected after considering, for example, compatibility with myeloma cells used for cell fusion. They include, for example, mouse, rat and hamster. Preferably, the mammal is mouse.

Further, splenic cells are preferably used as immune cells.

Myeloma cells used for the present invention include, for example, P3 (P3X63Ag8.653) (J. Immunol., 123,1548, 1978), p3-U1 (Current Topics in Micro-biology and Immunology, 81, 1-7,1978), NS-1 (Eur. J. Immunol., 6, 511-519, 1976), MPC-11 (Cell, 8, 405-415, 1976), Sp2/0-Ag14 (Nature, 276, 269-270, 1978), FO (J. Immunol. Meth., 35, 1-21, 1980), S194 (J. Exp. Med., 148, 313-323, 1978) and R210 (Nature, 277, 131-133, 1979). The myeloma cell is preferably P3 or p3-U1, more preferably P3.

Immune cells and myeloma cells can be fused, for example, by a method according to Milstein et. al. (Methods Enzymol., 73, 3-46, 1981). Specifically, cell fusion can be performed, for example, by mixing immune cells and myeloma cells in culture medium in the presence of a fusion promoter. Then, addition of culture medium and centrifugation can be appropriately repeated during cell fusion to produce hybridomas.

Culture media used for cell fusion include, for example, culture media usually used in cell fusion such as RPMI-1640 culture medium and MEM culture medium. Further, blood serum supplements such as fetal calf serum (FBS) can be suitably used together.

Temperature for cell fusion is preferably 25 to 37°C, and more preferably 30 to 37°C.

A mixing ratio of myeloma cells and immune cells is preferably about 1:1 to 1:10.

Fusion promoters may include, for example, polyethylene glycol (PEG) and Sendai Virus (HVJ). The fusion promoter is preferably PEG. The molecular weight of PEG can be suitably selected, and for example, the average molecular weight can be between about 1,000 and 6,000. The concentration of PEG in culture medium is preferably about 30 to 60% (W/V).

Auxiliary agents such as dimethyl sulfoxide can be suitably added to culture medium as desired.

Selection of the hybridoma of the present invention can be performed by culturing hybridomas obtained by cell fusion, for example, in common selection medium such as HAT culture medium, and using the limiting dilution method to conduct screening for, for example, on the basis of an indicator such as an antibody titer against a peptide consisting of an amino acid sequence represented by SSVLYGGPPSAA (SEQ ID NO:1) or a conjugate of the peptide and a pharmaceutically acceptable carrier. A culture period in HAT culture medium is a sufficient period for cells (non-fused cells) other than the hybridoma of interest to die, and usually can be several days to several weeks. The hybridoma of the present invention obtained in this way can be subcultured in common culture medium, and also can be stored for a long time in liquid nitrogen.

Methods of harvesting the monoclonal antibody of the present invention or an antibody binding fragment thereof include, for example, a method where hybridoma is cultured according to the conventional method to obtain monoclonal antibody and the like from the culture supernatant or a method where hybridoma is administered to a compatible mammal for proliferation and monoclonal antibody and the like is obtained from its ascitic fluid. Here, the former method is preferred for obtaining highly pure antibody while the latter method is preferred for producing a large amount of antibody.

Further, the monoclonal antibody of the present invention or an antibody binding fragment thereof can be purified to a high purity by methods such as salting-out, gel filtration and affinity chromatography.

The monoclonal antibody of the present invention or an antigen binding fragment thereof has an excellent therapeutic effect on inflammatory diseases in which histone is involved as described above. Therefore, according to another aspect of the present invention, provided is use of the monoclonal antibody of the present invention in manufacturing a therapeutic agent for inflammatory disease. In the above-mentioned method, inflammatory diseases are preferably inflammatory diseases in which histone is involved, more preferably acute inflammatory diseases, more preferably sepsis, renal ischemia reperfusion injury or renal failure, still more preferably sepsis, renal ischemia reperfusion injury or acute renal failure, still further preferably sepsis. In addition, the monoclonal antibody of the present invention or an antigen binding fragment thereof may be used as it is, or may be used as a pharmaceutical composition along with a pharmacologically acceptable additive. Therefore, according to one aspect of the present invention, provided is a pharmaceutical preparations for treating inflammatory diseases comprising the monoclonal antibody of the present invention or an antigen binding fragment thereof.

The therapeutic agent for treating sepsis of the present invention can be prepared, for example, by solving the monoclonal antibody of the present invention in injectable saline, injectable distilled water, an injectable buffer solution and the like. The composition for immunosuppression of the present invention may further contain a suitable solvent, a solubilizing agent, a preserving agent, a stabilizing agent, an emulsifying agent, a suspending agent, a soothing agent, a tonicity adjusting agent, a buffer, an excipient, a thickener, a coloring agent, a known carrier (various liposomes, polyamino acid carriers, synthetic macromolecules, naturally-occurring polymers and the like) and the like.

Further, according to another aspect of the present invention, provided is a method of treating inflammatory disease comprising administrating an effective amount of the monoclonal antibody of the present invention or an antigen binding fragment thereof. In this context, the term "treating" means alleviating established pathology. Furthermore, according to another aspect of the present invention, provided is a method of reducing risk of a subject for developing inflammatory disease comprising administering an effective amount of the monoclonal antibody of the present invention or an antigen binding fragment thereof to the subject. In the above-mentioned method, inflammatory diseases are preferably inflammatory diseases in which histone is involved, more preferably acute inflammatory diseases, more preferably sepsis, renal ischemia reperfusion injury or renal failure, still more preferably sepsis, renal ischemia reperfusion injury or acute renal failure, still further preferably sepsis.

Further, the monoclonal antibody of the present invention or an antigen binding fragment thereof can exert a prominent immunosuppressive effect. It is surprising that the above-mentioned monoclonal antibody or an antigen binding fragment thereof which may be used in the treatment of inflammatory diseases can exert an immunosuppressive effect. Therefore, according to one aspect, the monoclonal antibody of the present invention or an antigen binding fragment thereof is used as an immunosuppressive agent.

According to one aspect, the above-mentioned subjects are preferably a mammal, more preferably a human.

Further, the monoclonal antibody of the present invention or an antigen binding fragment thereof may be simultaneously or sequentially administered to a mammal in combination with other agents used for inflammatory diseases.

Further, the monoclonal antibody of the present invention or an antigen binding fragment thereof can be administered systemically or locally. Specific methods of administration include infusion, intravenous injection, intramuscular injection, subcutaneous injection, intradermal injection, oral administration, transmucosal administration and transdermal administration.

Furthermore, the effective amount of the monoclonal antibody of the present invention or an antigen binding fragment is not particularly limited, and can be suitably determined by the person skilled in the art depending on species, nature, sex, age, symptoms and the like of the subject. For example, such effective amounts include one or several doses of 0.05 to 40 mg/kg weight/day, preferably 2 to 10 mg/kg weight/day.

### Examples

In the followings, the present invention will be specifically described with reference to Examples, but the present invention is not limited to these Examples.

### Example 1: Production of monoclonal antibody (SSVmAb)

### Production of an antigenic substance

For an antigenic substance, a conjugate of a peptide consisting of an amino acid sequence represented by SEQ ID NO:1 and KLH were used.

In preparation of the antigenic substance, first, a peptide consisting of an amino acid sequence represented by SEQ ID NO:1 was synthesized by the Fmoc peptide solid phase synthesis method (a manufacturing instrument; Applied Biosystems ABI 430). A conjugate of the above-mentioned peptide and KLH (SIGMA) was synthesized by stirring 5 mg of the above-mentioned peptide, about 20 mg KLH and 30 µg glutaraldehyde (Katayama Chemical Industries Co., Ltd.) in phosphate buffer (pH 8.0) at room temperature for about 6 hours.

### Production of hybridoma

### Immunization

Suspension (the concentration of the antigen: 0.25 mg/mL) was obtained by mixing 0.8 mL of a solution in which the antigenic substance was dissolved in PBS (the concentration of the antigenic substance: 0.5 mg/mL) and 0.8 mL complete Freund's adjuvant (Wako Pure Chemical Industries, Ltd.). Then, 0.2 mL of this suspension was intraperitoneally administered to a BALB/c mouse. This suspension in the same amount was further administered to the mouse every two weeks. Then, 16 weeks after the administration was started, 0.2 mL of a solution in which the antigen was dissolved in PBS (the concentration of the antigen: 600 to 1000 mg/mL) was intraperitoneally administered to the mouse as a final dose. Note that blood was withdrawn via a vein at the back of the eye when administering, and an antibody titer was measured by ELISA. Four days after the last administration, exsanguination was performed, and the blood obtained was centrifuged (2000 rpm, 20 minutes) to obtain antiserum, which was used as control antiserum in the following experiments. Further, after exsanguination, spleen was removed from the rat, and the splenic cells obtained were used in cell fusion as follows.

### Cell fusion

The above-mentioned splenic cells and myeloma cells (P3X63-Ag.8.653) were mixed at splenic cells: myeloma cells = 10:1 to 10, and centrifuged (1500 rpm, 5 minutes). After centrifugation, the supernatant was removed by using an aspirator, and 1 mL polyethylene glycol 4000 (50% PBS solution) at 37°C was added over 1 minute to the cell pellet obtained to form a mixed liquid. After allowing this mixed liquid to stand at 37°C for 1 minute, 1 mL IMDM culture medium at 37°C was each added every 30 seconds (total 9 mL), and then centrifuged (1500 rpm, 5 minutes). After centrifugation, the supernatant was removed by suction, and an appropriate amount of 15% FCS (JRH BIOSCIENCES) containing IMDM (GIBCO) culture media at 37°C was added. The suspension obtained was dispensed into a 96 well culture plate in an amount of 100 mL for each, and cultured for one day in an incubator at 37°C/5% CO₂. Further, 100 mL HAT culture medium (HAT powder (HAT MEDIA SUPPLEMENT (x50), SIGMA) was dissolved in 10 mL serum free IMDM culture medium, which was then diluted 50 times with 10% FCS containing IMDM culture medium) was added, and cultured in an incubator at 37°C/5% CO₂. HAT culture medium was replaced every 2 to 3 days, and after 10 days, it was switched to HT culture medium (HT powder (HT MEDIA SUPPLEMENT, SIGMA) was dissolved in 10 mL serum free IMDM culture medium, which was then diluted 50 times with 10% FCS containing IMDM culture medium.), and cultured in an incubator at 37°C/5% CO₂ for three days. After that, the culture medium (HT culture medium) was replaced every 2 to 3 days. After verifying cell growth under a microscope, the culture supernatants (about 100 mL) were collected. Using the culture supernatants, screening of hybridoma was performed by measuring antibody titers.

### Screening of hybridoma cells

### Measurement of antibody titer

A buffer solution containing the above-mentioned antigenic substance (5 mg) (Baicarbonate buffer: 100 mM NaHCO₃-NaOH, pH 9.2 to 9.5, the concentration of the peptide: 1 µg/mL) was added to a 96 well flat bottom plate in an amount of 50 µL per well, and allowed to stand for coating at room temperature for 2 hours. The plate was washed 3 times with wash buffer (PBST), and then blocking buffer (3% skim milk 1% BSA, PBS) was added in an amount of 200 to 250 µL/well to react at 4°C for one full day, and then washed 3 times. Then the culture supernatant of hybridoma was added in an amount of 100 µL/well, which was allowed to react at 37°C for 4 hours or at 4°C for one full day. After the plate was washed 3 times, biotin-labeled anti-mouse IgG (SIGMA) diluted 10000 times with dilution buffer (10 mM Tris-HCl (pH 8.0), 0.9% (W/V) NaCl, 0.05% (W/V) Tween 20) was added in an amount of 50 µL/well, which was allowed to react at room temperature for 2 hours. After washing was performed 6 times, alkaline phosphatase labeled Streptaridin diluted 1000 times with dilution buffer was added in an amount of 50 µL/well, which was allowed to react at room temperature for 1 to 2 hours. Then washing was performed 6 times, and fluorescent substrate buffer (Attophos substrate buffer, Roche Diagnostics K.K.) was added in an amount of 50 µL/well, and the plate was shaded to allow fluorescence to develop. Fluorescence intensity was measured in CytoFluorII (PerSeptive Biosystems).

### Screening of hybridoma

To the wells which showed a positive result in the above-mentioned measurement of antibody titer (1x10⁵ cells/mL), 15% FCS 10% HCF (Hybridoma cloning factor, ORIGIN) containing IMDM culture medium was added, which was dispensed in a 96 well culture plate in an amount of about 200 cells/well, and cultured in an incubator at 37°C 5% CO₂. Then antibody titers were measured as described above, hybridomas showing a high antibody yield were selected.

Limiting dilution was further performed so that the selected hybridoma was diluted to 0.5 to 1 cell/well with 15% FCS 10% HCF containing IMDM culture medium. After culturing in an incubator at 37°C/5% CO₂ for about three to four days, antibody titers were measured as described above to select hybridomas showing a high antibody yield. Limiting dilution was further repeated to obtain hybridomas which produce monoclonal antibody against the above-mentioned antigenic substance. Among these, the hybridoma with the highest antibody titer was selected and designated as Mouse-Mouse hybridoma SSV-C 93-3.

### Acquisition of monoclonal antibody

Hybridoma Mouse-Mouse hybridoma SSV-C 93-3 was cultured using 15% FCS containing RPMI culture medium (1x10⁶ cells/mL). Then, hybridoma culture medium was collected, and filtered through a filter in order to remove dead cell debris. Then, ammonium sulfate was added to the culture supernatant to a final concentration of 40%, and stirred at 40°C for 1 hour. Then, centrifugation (3000 g, 30 minutes, 4°C) was performed, and the supernatant was discarded to collect precipitate. The precipitate was dissolved in a volume of PBS equivalent to a 1/10 amount of the above-mentioned culture supernatant, and dialyzed against PBS overnight.

Then, the above-mentioned precipitate was diluted twice with 20 mM sodium phosphate buffer (pH 7.0), and loaded onto a HiTrap NHS activated column along with 1 M Tris-HCl buffer. Then, antibody was eluted with a 0.1 M glycine HCl solution (pH 2.7), and collected in fraction tubes.

### Test Example 1: Identification of SSVmAb isotype

In order to identify the isotype of the monoclonal antibody (SSVmAb) of Example 1, isotype identification tests were performed using Mouse Monoclonal Antibody Isotyping Reagents (SIGMA).

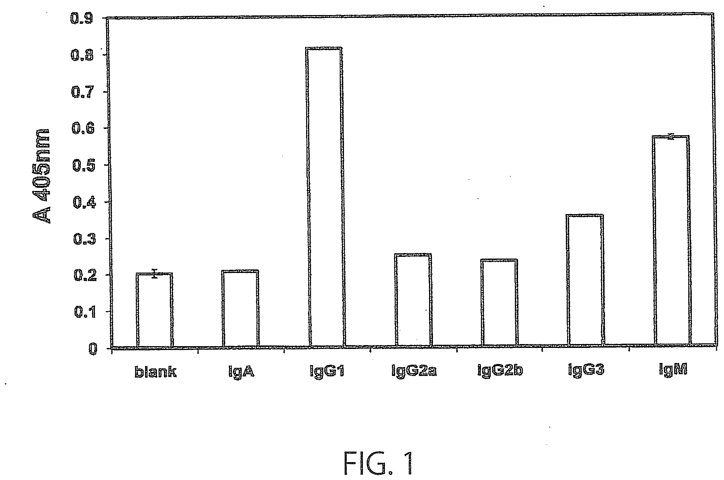

The results are shown in Fig. 1, indicating that IgG1 showed the highest value.

Further, when mouse IgG1 (eBioscience) and the monoclonal antibody (SSVmAb) of Example 1 were reduced by 2-mercaptoethanol and analyzed by SDS-PAGE, the bands corresponding to a heavy chain and a light chain were observed at similar positions (50 KD, 25 KD) for both. On the other hand, similar bands were not observed, when a similar experiment was conducted using mouse IgM (eBioscience) instead of mouse IgG1.

From Fig. 1 and the results of SDS-PAGE, the isotype of the monoclonal antibody (SSVmAb) of Example 1 was determined to be IgG1.

### Test Example 2: Determination of the affinity of SSVmAb for core histone

WO2006/025580 has reported the monoclonal antibody (16G9mAb) produced by hybridoma 16G9 (Deposition Number FERM BP-10413) as an anti H1 monoclonal antibody which can be used for immunosuppression and which binds to a peptide consisting of an amino acid sequence represented by SEQ ID NO:1.

Therefore, using the antibody (16G9mAb) described in WO2006/025580 as a Reference Example 1, the affinity for an antigen was compared with that of the monoclonal antibody (SSVmAb) of Example 1.

For an antigen, histone H1, which is an antigen of Reference Example 1 (16G9mAb), and core histone H2A, H2B, H3 and H4, which are histone H1 antigen analogs, were selected.

The Affinities between histone H1 or core histone and SSVmAb were determined by ELISA.

A 96 well microplate was coated with histone H1, H2A, H2B, H3 or H4. Each histone used was dissolved in 100 mM sodium carbonate buffer (pH 9.3). The plate was washed with PBS-tween 20 (0.05%), and blocked with 3% skim milk and 1% BSA for 1 hour. To each well, 5 µg/mL SSVmAb was added, and incubated for 1 hour. Bound SSVmAb was detected using peroxidase (HRP) conjugated anti mouse IgG1 Ab (SIGMA), and incubated for 1 hour. Bound SSVmAb was detected using the ABTS [2,2'-azino-bis(3-ethylbenzothiazoline-sulfonic acid)] substrate solution, and absorbance at 405 nm was measured using Multiskan Ascent (Thermo Fisher Scientific Inc., Waltham, MA).

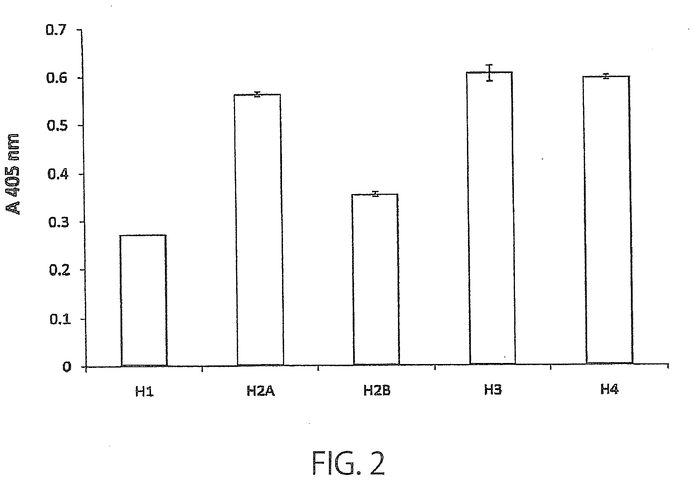

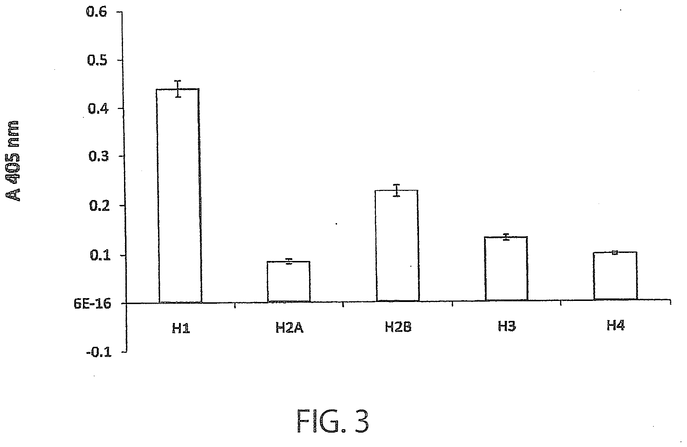

The results are shown in Figs. 2 and 3.

As shown in Fig. 2, for Example 1 (SSVmAb), the affinities for histone H2A, H2B, H3 or H4 were higher than the affinity for histone H1.

On the other hand, as shown in Fig. 3, for Reference Example 1 (16G9), the affinity for histone H1 was higher than the affinities for histone H2A, H2B, H3 and H4.

### Test Example 3: MLR tests

Spleen lymphocytes from a naive DA rat (responsive cells) and spleen lymphocytes from a LEW rat treated with mitomycin-C (Kyowa Hakko Kogyo Co., Ltd.) (stimulated cells) were used. The responsive cells were adjusted to 5x10⁵ cells/mL with 10% FCS-RPMI culture medium, and the stimulated cells were adjusted to 8x10⁶ cells/mL with 10% FCS-RPMI culture medium. After plating the responsive cell suspension and the stimulated cell suspension in an amount of 100 µL to a 96 well round-bottom plate (Nunc Brand Products) respectively, the monoclonal antibody 16G9mAb of Reference Example 1 (0.1, 2, 4, or 6 µg/mL/well) or the monoclonal antibody SSVmAb of Example 1 (4 µg/mL/well) was added at the start of mixed culture, and cultured for 3.5 days or longer under the conditions of 37°C, 5% CO₂/95% air. In addition, an immunosuppressive agent tacrolimus (FK506: Fujisawa Pharmaceutical Co., Ltd., 1 nM/well) was added as a positive control. Further, 10 µL bromo deoxyuridine (BrdU) was added 15 hours before the end of culture. Then the proliferation potential of the cells treated with the immunosuppressive agent was measured using BrdU labeling & detection kit III (Roche Diagnostics K.K.) using the amount of BrdU incorporated into cellular DNA as an indicator.

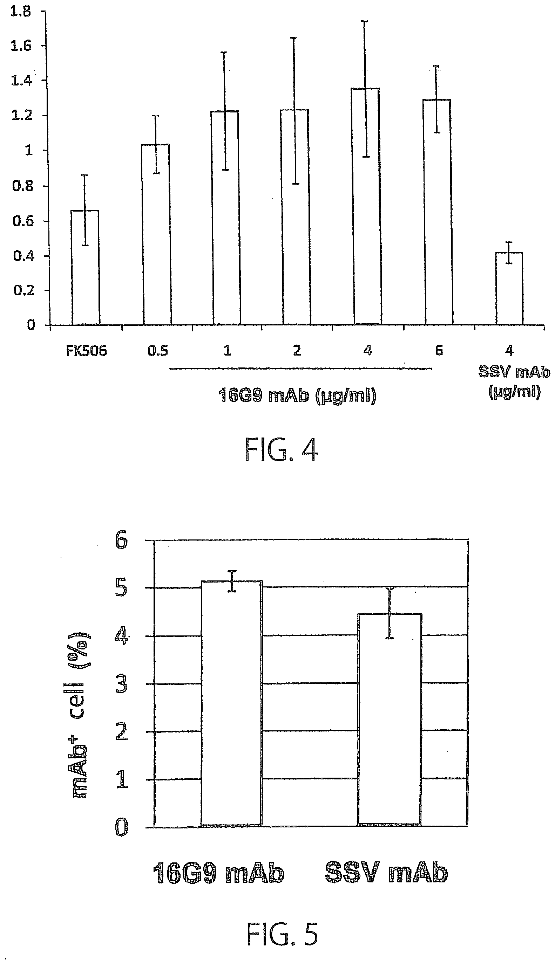

The results are shown in Figs. 4.

For Example 1 (SSVmAb), the absorbance which indicates the amount of incorporated BrdU was lower than that of Reference Example 1 (16G9mAb) and tacrolimus (FK506). In particular, when the absorbance 0.552 ± 0.114 (mean ± S.E.) of Example 1 (SSV mAb) and the absorbance 1.351 ± 0.389 (mean ± S.E.) of Reference Example 1 (16G9mAb) where the same amount was added (4 µg/mL/well) were compared, the mean of Example 1 was about 41% of that of Reference Example 1.

### Test Example 4 : Determination of the reactivity of the monoclonal antibody (SSVmAb) with T cells

Using the following approach, spleen was removed from a C57BL/6 mouse (5 weeks old, female, CHARLES RIVER LABORATORIES JAPAN, INC.) to prepare whole splenic cells.

First, in a 5 ml culture dish (BD Bioscience FALCON 351007) into which 5 ml RPMI 1640 culture medium (Sigma-Aldrich, R-8758) was transferred, spleen was disentangled well with scissors for dissection and forceps to suspend the splenic cells, which were then transferred to a 15 ml centrifuge tube (BD Bioscience FALCON 352096). Then, the 5 ml dish was washed several times with phosphate-buffered saline (PBS, Invitrogen, 20012-027), and these were also added to the foregoing cell suspension and allowed to stand, and then the supernatant was collected in another 15 ml centrifuge tube. In addition, 5 ml RPMI 1640 culture medium was also added to the residual insoluble spleen tissue again and allowed to stand, and then only the supernatant was collected, which was combined with the above-mentioned cell suspension to perform centrifugation at 1,500 rpm for 5 min. To the collected cells, added was 2 ml lysis buffer (150 mM NH₄Cl/15 mM NaHCO₃/0.1 mM EDTA-Na₂, pH 7.3) and hemolyzed by tapping, and then 10 ml PBS was added. After washed 3 times by centrifugation at 1,500 rpm for 5 min, whole splenic cells were obtained.

Next, according to the approach described below, whole T cells were purified from the above splenic cells by magnetic sorting (MACS) using Pan T Cell Isolation Kit, mouse (Miltenyi Biotec, 130-090-861).

First, the splenic cells were suspended at a ratio of 5x10⁷ cells/200 µl in MACS buffer (0.5% bovine serum albumin (BSA, NACALAI TESQUE, INC., 08777-36)/PBS), to which 50 µl Biotin-antibody cocktail/5x10⁷ cells was added and incubated at 4°C for 10 min. After this was suspended in 150 µl MACS buffer/5x10⁷ cells, 100 µl anti-biotin micro beads/5x10⁷ cells was added and incubated at 4°C for 15 min. To this, MACS buffer (10 ml) was added and washed by centrifugation at 1500 rpm for 5 min, and then the recovered cells were suspended in 500 µl MACS buffer. After a MACS column (MS column, Miltenyi Biotec, 130-042-201) was placed in a magnet (MiniMACS separation Unit, Miltenyi Biotec, 130-090-312) and the column was equilibrated with 500 µl MACS buffer, the above-mentioned cell suspension was loaded. The 500 µl flow through fraction and the subsequent column washing fraction (1.5 ml) with MACS buffer were collected to give a purified unstimulated T cells (about 97% pure).

The reactivity of the above unstimulated with 16G9 mAb or SSV mAb was analyzed by flow cytometry (FACS).

First, after each T cell sample (1x10⁶ cells) was suspended in 89 µl FACS buffer (0.5% FBS/PBS/0.02% NaN₃), 1 µg anti-mouse CD16/32-blocks Fc binding (eBioscience, 14-0161-85) was added and incubated at 4°C for 20 min. To this, 10 µl 16G9 mAb or SSV mAb (100 µg/ml) was added as a primary antibody and incubated at 4°C for 60 min. After the cells were washed by centrifugation twice with FACS buffer, a 100 µl volume of a secondary antibody (Biotin-conjugated anti-mouse IgM mAb (eBioscience, 13-5780-85) or Biotin-conjugated rat anti-mouse IgG1 mAb (BD Biosciences, 553441), 1 µg/ml each) was added and incubated at 4°C for 30 min. After the cells were again washed by centrifugation twice with FACS buffer, 100 µl Streptavidin-PE-Cy7 (BD Biosciences, 556463, 1 µg/ml) was added, to which FITC-conjugated rat anti-mouse CD3 mAb (BDBiosciences, 553062) was added to a final concentration of 1 µg/ml and incubated at 4°C for 30 min. After washed by centrifugation twice with FACS buffer and filter-treated with a 40 µm cell strainer (BD Bioscience, FALCON 352340), each sample was subjected to a FACSCalibur flow cytometer and CellQuest software (BD Bioscience) to analyze the number of 16G9 mAb or SSV mAb positive/CD3 positive T cells.

The results are shown in Figs. 5.

When Reference Example 1 (16G9 mAb) was compared with Example 1 (SSV mAb), no significant difference was observed for the reactivity with CD3 positive T cells, and these antibodies showed comparative reactivity (student t-test, p < 0.05).

### Test Example 5: Identification of a target for down-regulation by SSV mAb

Seven candidate proteins which may be down-regulated by Example 1 (SSV mAb) were identified by the proteome analysis.

Then among the 7 candidate proteins, ATP synthase was determined to be a target antigen of Example 1 (SSV mAb) by the method described below.

First, T cells from a Balb/c mouse having mitochondria ATP synthase knocked down were obtained using Accell siRNA kit from Thermo Fisher Scientific Inc.

Next, according to the method in Test Example 2, MLR tests were performed using Example 1 (SSV mAb) as a test substance using the T cells obtained.

In the test, Isotype IgG1 (eBioscience) was used as a control reagent. In addition, a similar test was performed using T cells from a mouse not having the ATP synthase knocked down as a control test.

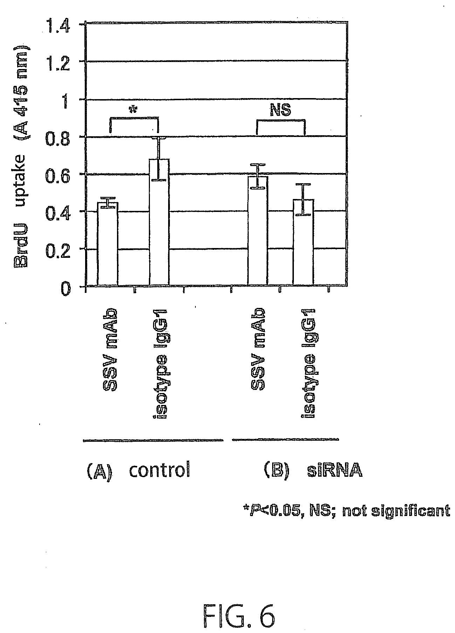

The results are shown in Figs. 6A and 6B.

As shown in Fig. 6A, when the ATP synthase was not knocked down, Example 1 (SSV mAb) significantly inhibited cell growth as compared with Isotype IgG1.

On the other hand, as shown in Fig. 6B, when the ATP synthase was knocked down, no significant difference was observed for cell growth inhibition between Example 1 (SSV mAb) and Isotype IgG1.

Fig. 6A and 6B suggests that the immunosuppressive activity of SSV mAb is decreased by knocking down the ATP synthase, and that SSV mAb down-regulates the activity of the ATP synthase upon immunosuppression.

### Test Example 6: Identification of the sequence for the variable regions of the light and heavy chains of SSV mAb

### Synthesis of hybridoma cDNA

Total RNA was prepared from the 1.6x10⁷ cells of hybridoma obtained in Test Example 1 (Mouse-Mouse hybridoma SSV-C 93-3) using FastPure RNA Kit (TaKaRa). Using Poly (A)⁺ Isolation Kit from Total RNA (NIPPON GENE), 240 µg of total RNA was prepared from mRNA. Ethanol precipitation was performed using Etachinmate (NIPPON GENE) to precipitate mRNA. After washed with 75% ethanol, mRNA was dried. To this, 10 µL RNase free water was added to dissolve mRNA. The mRNA solution obtained was stored at -80°C. Using SMARTer RACE cDNA Amplification Kit (Clontech), cDNA for 5'-RACE was synthesized from 1 µg SSV hybridoma mRNA. The cDNA solution obtained was stored at -20°C.

### Identification of the complementarity determining regions (CDR) in the light and heavy chains of SSV mAb

Based on the base sequence of the mouse IgG1 heavy chain constant region, a primer, 5'-CAC CAT GGA GTT AGT TTG GGC AGC AG-3' (SEQ ID NO:12) was produced. Based on the base sequence of the mouse light κ chain constant region, a primer, 5'-CAC GAC TGA GGC ACC TCC AGA TG-3' (SEQ ID NO:13) was produced. Using a respective primer and Universal Primer A Mix (a primer included in SMARTer RACE cDNA Amplification Kit), 5'-RACE was performed using cDNA as a template. For the RACE reaction, Advantage2 PCR Kit (Clontech) was used. The reaction mixture was subjected to agarose electrophoresis, and a heavy chain 5'-RACE product of about 600 bp and a light chain 5'-RACE product of about 550 bp were purified from the gel using E.Z.N.A. Gel Extraction Kit (OMEGA bio-tek). This was linked to pGEM-T Easy Vector (Promega), with which Competent high E.coli DH5α (TOYOBO) was transformed. From the resulting transformant, the plasmid was prepared using E.Z.N.A. Plasmid Miniprep KitI (OMEGA bio-tek). Using the prepared plasmid as a template, cyclical reactions were performed using BigDye Terminator v3.1 Cycle Sequencing Kit (Applied Biosystems).

Next, the base sequences of the light and heavy chain variable regions were analyzed using a DNA sequencer (Applied Biosystems).

As a result, the base sequence of the light chain variable region was found to be represented by Position 67 to Position 384 of SEQ ID NO:5.

Further, the base sequence of the heavy chain variable region was found to be represented by Position 58 to Position 423 of SEQ ID NO:10.

Based on the position of FR (constant region) 1 as determined by the translation initiation codon and the method according to Kabat el al., the followings were estimated: Position 1 to Position 66 of SEQ ID NO:5 corresponds to the base sequence of the light chain signal peptide, and Position 1 to Position 57 of SEQ ID NO:10 corresponds to the base sequence of the heavy chain signal peptide.

Next, the amino acid sequences of the variable regions of the light and heavy chains were estimated from the base sequences obtained, and CDR regions was identified in accordance with the method of Kabat et al.

As a result, the amino acid sequence of the light chain variable region was found to be represented by Position 23 to Position 128 of SEQ ID NO:6. Here, Position 1 to Position 22 of SEQ ID NO:6 corresponds to the amino acid sequence of the light chain signal peptide.

Further, in the amino acid sequence of the light chain variable region, the followings were determined: CDR1 is represented by RASSSVSYMH (SEQ ID NO:2), CDR2 is represented by ATSNLAS (SEQ ID NO:3), and CDR3 is represented by QQWSSNPWT (SEQ ID NO:4).

Furthermore, the amino acid sequence of the heavy chain variable region was found to be represented by Position 20 to Position 141 of SEQ ID NO:11. Here, Position 1 to Position 19 of SEQ ID NO:11 corresponds to the amino acid sequence of the heavy chain signal peptide.

Moreover, in the amino acid sequence of the heavy chain variable region, the followings were determined: CDR1 is represented by GYNMN (SEQ ID NO:7), CDR2 is represented by NINPYYGSTSYNQKFKG (SEQ ID NO:8), and CDR3 is represented by SPYYSNYWRYFDY (SEQ ID NO:9).

### Test Example 7:Evaluation of Therapeutic Action against Sepsis

BALB/c mice (bodyweight of 20 g to 30 g when starting the experiments, n=6) were raised in a plastic cage under SPF and thermo-hydrostat (22 ± 1°C, 55±5%) conditions under 12-hour light-dark cycle. Foods and water were supplied ad libitum. Lipopolysaccharide (LPS)-induced lethal sepsis which is generally employed as an experimental sepsis model was induced by intraperitoneally administering LPS at a dose of 40 mg/kg. SSV mAb dissolved in physiological saline was intraperitoneally administered totally 3 times, i.e., at 30 minutes before, 6 hours after and 12 hours after the intraperitoneal administration of LPS, in an amount of 100 µg/dose in terms of the amount of SSV mAb. The survival of the animals thereafter was tested. To the control group, IgG (SIGMA) dissolved in physiological saline was administered in place of SSV mAb in the same manner.

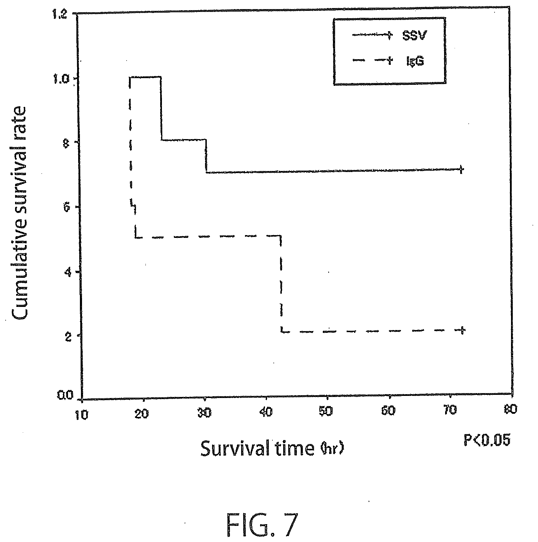

The results are shown in Fig. 7. The survival rate at 70 hours after administration of LPS (induction of sepsis) was 20% (cumulative survival rate: 0.2) in the control group (IgG) and 70% (cumulative survival rate: 0.7) in the SSV mAb-administered group. The survival rate at 70 hours after administration of LPS (induction of sepsis) of the SSV mAb-administered group (SSV) was about 3.5 times of that of the control group, and was significantly higher than the control group (p<0.05).

### Test Example 8:Evaluation of Therapeutic Action against Inflammatory Disease 8-1

BALB/c mice (bodyweight of 20 g to 30 g when starting the experiments, n = 10) were raised in a plastic cage under SPF and thermo-hydrostat (22 ± 1°C, 55 ± 5%) conditions under 12-hour light-dark cycle. Foods and water were supplied ad libitum. Then, by intraperitoneally administering LPS to the mice at a dose of 40 mg/kg, lipopolysaccharide (LPS)-induced lethal sepsis as an inflammatory disease was induced in the same manner as Test Example 7. SSV mAb dissolved in physiological saline was intraperitoneally administered totally 3 times, i.e., at 30 minutes before, 6 hours after and 12 hours after the intraperitoneal administration of LPS, in an amount of 100 µg/dose in terms of the amount of SSV mAb. The survival of the animals thereafter was tested. To the control group, IgG (SIGMA) dissolved in physiological saline was administered in place of SSV mAb in the same manner.

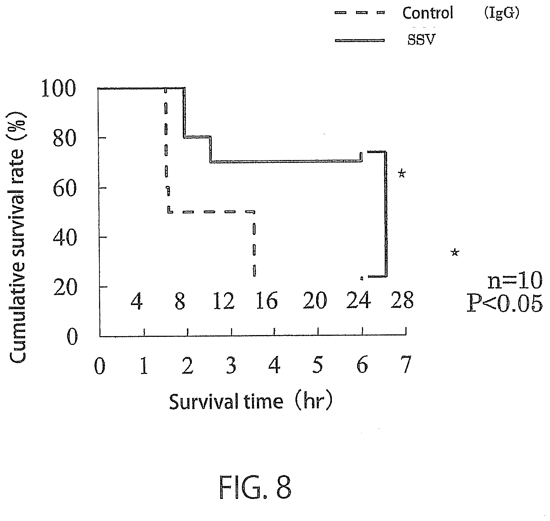

The results are shown in Fig. 8. The survival rate at 24 hours after administration of LPS (induction of sepsis) was 20% in the control group (IgG) and 70% in the SSV mAb-administered group. The survival rate at 70 hours after administration of LPS (induction of inflammation) of the SSV mAb-administered group (SSV) was about 3.5 times of that of the control group, and was significantly higher than the control group (p < 0.05).

### 8-2: Measurement of Histone concentration in Serum and Inflammatory Tissue

In Test Example 8, heart-derived blood or lung tissue was obtained from control group and SSV mAb-administered group under general anesthesia according to the method conducted by Hasegawa et al. (Surg Res. 2012 May 1;174(1):136-41.). The concentration of histone H1, histone H3 and H4 of the obtained samples were measured by using a commercially available measurement kit using ELISA.

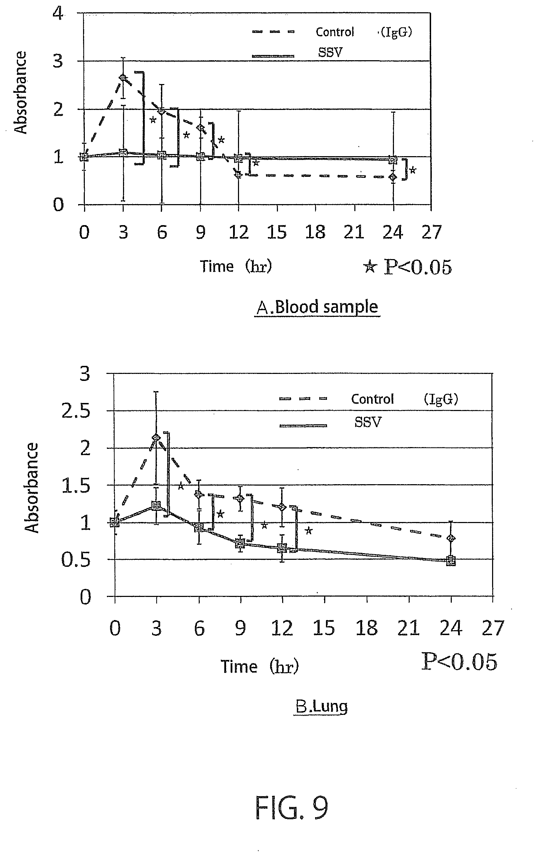

The results of measuring histone H1 concentration are as shown in Fig. 9A (blood-derived serum) and Fig. 9B (lung tissue).

The concentration of histone H1 in blood sample from the SSV mAb-administered group was almost kept constant without increase during the test. On the other hand, in the concentration of histone H1 in blood sample from the control group, changes were observed during the test.

In addition, it is confirmed that the concentration of histone H1 in inflammatory tissue (lung) from the SSV mAb-administered group was more significantly decreased than that from the control group.

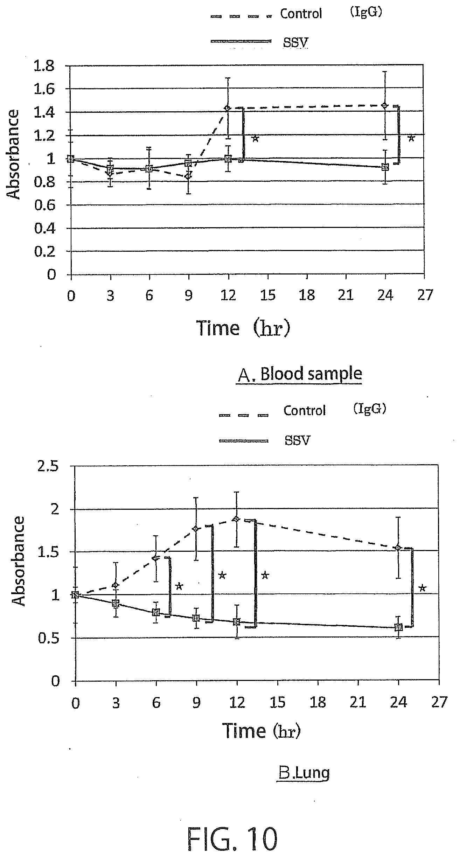

The results of measuring histone H3 concentration are as shown in Fig. 10A (blood-derived serum) and Fig. 10B (lung tissue).

A tendency that the concentrations of histone H3 in blood sample and inflammatory tissue (lung) from the SSV mAb-administered group were lower than the control group was observed.

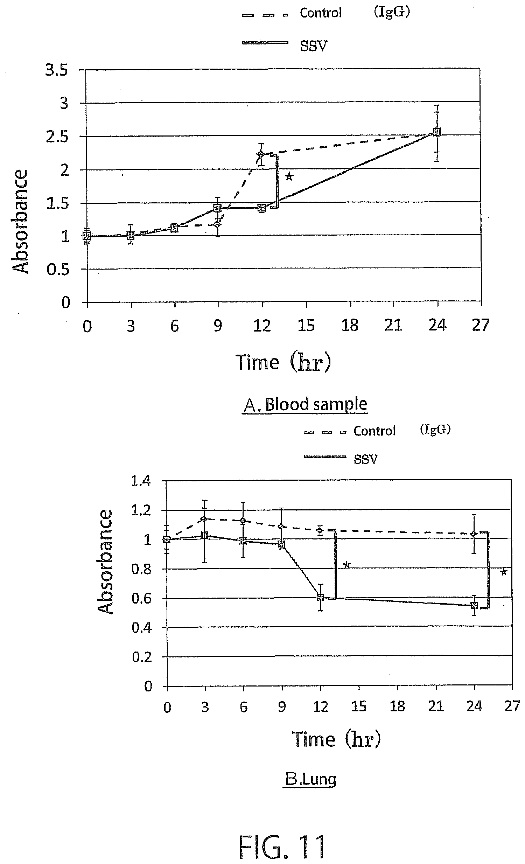

The results of measuring histone H4 concentration are as shown in Fig. 11A (blood-derived serum) and Fig. 11B (lung tissue).

The concentrations of histone H3 in blood sample and inflammatory tissue (lung) from the SSV mAb-administered group were found to tend to be lower than the control group.

After the end of the tests, binding assays of SSV mAb with histone H1, histone H3 and histone H4 were carried out by ELISA in the same manner as Test Example 2.

As a result, SSV mAb was found to have a binding capacity with histone H1, histone H3 and histone H4 *in vitro.*

### 8-3: Staining Test of Lung Tissue

After the end of Test Example 8, lung tissue sections were each obtained from rats of control group and SSV mAb-administered group, and stained by using hematoxylin and eosin stain (from Wako Pure Chemical Industries, Ltd.) according to the method conducted by Hasegawa et al. (Surg Res. 2012 May 1;174(1):136-41.). Next, micrographs of the obtained samples were taken.

As a reference, micrographs of lung tissue slices from healthy rats were taken in the same manner.

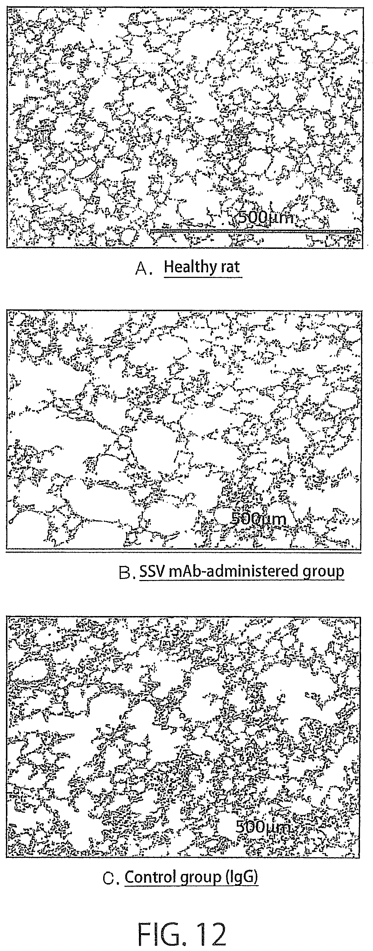

The results were as shown in Fig. 12A-C.

Inflammation in lungs was not observed in Fig. 12A (healthy rat) and Fig. 12B (SSV mAb-administered group). On the other hand, in Fig. 12C (control group), inflammation in lungs was observed.

### 8-4: Evaluation of Congestion, Edema, Inflammation and Bleeding

After the end of Test Example 8, scores of congestion, edema, inflammation and bleeding were evaluated according to the method of Murakami et al. (Shock, 18 (2002), p. 236). In particular, magnified images of 24 visual fields under a microscope were subjected to observation. The degrees of deterioration of congestion, edema and inflammation in the SSV mAb-administered group and the control group were evaluated on a 5-point scale ranging from 0 to 4 (4 is the highest deterioration).

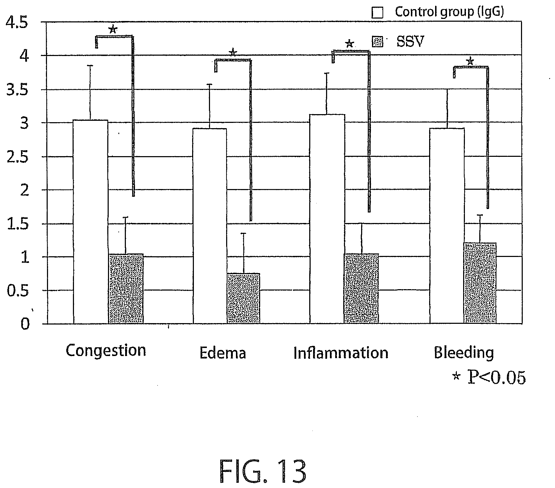

The results were as shown in Fig. 13 (mean score ± standard deviation).

In either of congestion, edema, inflammation and bleeding, the scores of the SSV mAb-administered group was shown to be significantly lower than that of the control group.

### 8-5: Measurement of Inflammation-associated Cytokine

In Test Example 8, serum was obtained from venous blood of the control group and the SSV mAb-administered group every 3 hours. Then, the concentrations of inflammatory cytokine (TNF-α, IL-1β, IL-6) and inhibitory cytokine (IL-10) in obtained samples were measured by using a commercially available measurement kit using ELISA.

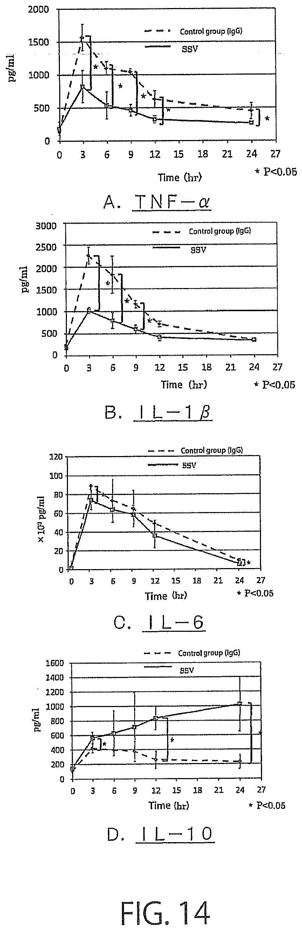

The results were as shown in Fig. 14A-D.

As shown in Fig. 14A-C, the values of inflammatory cytokine TNF-α, IL-1β and IL-6 of the SSV mAb-administered group were significantly decreased compared to the control group.

On the other hand, as shown in Fig. 14D, the value of inhibitory cytokine IL-10 of the SSV mAb-administered group was significantly increased compared to the control group.

The above-mentioned results also confirmed that inflammation in the SSV mAb-administered group is suppressed compared to the control group.

### Test Example 9: Check Test 1 for Therapeutic Effect against Acute Renal Failure due to Renal Ischemia Reperfusion Injury

Right kidney was removed from male Wistar rat (n=4) under general anesthesia, and then left kidney of the rat was subjected to ischemia reperfusion with vascular clip to prepare a model. For SSV mAb-administered group, SSV mAb was then administered in an amount of 10 mg/kg 30 minutes before preparing the model and immediately after the reperfusion
For control group, IgG (SIGMA) was administered in place of SSV mAb in the same manner. Then, urea nitrogen (BUN) and creatinine (Cr) in serum at 24 hours after preparing the model were measured and the nephropathy level was evaluated. Further, the rats were subjected to autopsy after the end of the test, and histological evaluation was performed.

The results were as shown in Table 1.

**Table 1**

| Group | BUN (mg/dL) | Cr (ml/min/kg) |
|---|---|---|
| Control | 217 ± 33 | 4.0 ± 0.3 |
| SSV mAb | 170 ± 31 | 3.9 ± 0.6 |

| | | |
|---|---|---|
| mean ± standard deviation | | |

The BUN and Cr values of the SSV mAb-administered group were significantly lower than that of the control group. Further, histological evaluation of kidney found that the state of the SSV mAb-administered group was better than that of the control group.

### Test Example 10: Check Test 2 for Therapeutic Effect against Acute Renal Failure due to Renal Ischemia Reperfusion Injury

Test was carried out in the same manner as Test Example 10 except that male Wistar rats (n = 4-6) were used and except that, for SSV mAb-administered group, SSV mAb was administered in an amount of 10 mg/kg 30 minutes before preparing the model and 6 hours after the reperfusion.

For control, IgG (SIGMA) was administered in place of SSV mAb in the same manner. Then, the concentrations of urea nitrogen (BUN) and creatinine (Cr) in serum at 24 hours after preparing the model were measured and the nephropathy level was evaluated. Further, the rats were subjected to autopsy after the end of the test, and histological evaluation was performed.

The results were as shown in Table 2.

**Table 2**

| Group | BUN (mg/dL) | Cr (ml/min/kg) |
|---|---|---|
| Control | 170 ± 18 | 4.3 ± 0.2 |
| SSV mAb | 137 ± 20 | 3.7 ± 0.5 |

| | | |
|---|---|---|
| mean ± standard deviation | | |

The BUN and Cr values of the SSV mAb-administered group were significantly lower than that of the control group. Further, histological evaluation of kidney found that the state of the SSV mAb-administered group was better than that of the control group.

## Claims

1. A therapeutic agent for use in a treatment of an inflammatory disease, which comprises a monoclonal antibody or an antigen binding fragment thereof which binds to a peptide comprising an amino acid sequence represented by SSVLYGGPPSAA (SEQ ID NO:1) or a conjugate of the peptide and a pharmaceutically acceptable carrier,
wherein the monoclonal antibody or an antigen binding fragment thereof comprises a light chain variable region comprising CDR1 consisting of an amino acid sequence represented by RASSSVSYMH (SEQ ID NO:2), CDR2 consisting of an amino acid sequence represented by ATSNLAS (SEQ ID NO:3), and CDR3 consisting of an amino acid sequence represented by QQWSSNPWT (SEQ ID NO:4), and a heavy chain variable region comprising CDR1 consisting of an amino acid sequence represented by GYNMN (SEQ ID NO:7), CDR2 consisting of an amino acid sequence represented by NINPYYGSTSYNQKFKG (SEQ ID NO:8), and CDR3 consisting of an amino acid sequence represented by SPYYSNYWRYFDY (SEQ ID NO:9),
wherein the light chain variable region of the monoclonal antibody or an antigen binding fragment thereof comprises an amino acid sequence represented by Position 23 to Position 128 of SEQ ID NO:6, and
wherein the heavy chain variable region of the monoclonal antibody or an antigen binding fragment thereof comprises an amino acid sequence represented by Position 20 to Position 141 of SEQ ID NO:11.

2. The therapeutic agent for use according to claim 1, wherein histone is involved in the inflammatory disease.

3. The therapeutic agent for use according to claim 1 or 2, wherein the inflammatory disease is acute inflammatory disease.

4. The therapeutic agent for use according to any one of claims 1 to 3, wherein the inflammatory disease is selected from sepsis, renal ischemia reperfusion injury and renal failure.

5. The therapeutic agent for use according to any one of claims 1 to 4, wherein the inflammatory disease is sepsis.

6. The therapeutic agent for use according to any one of claims 1 to 5, wherein the pharmaceutically acceptable carrier is keyhole limpet hemocyanin, ovalbumin or bovine serum albumin.

7. The therapeutic agent for use according to any one of claims 1 to 6, wherein the monoclonal antibody or an antigen binding fragment thereof can down-regulate an ATP synthase activity.

8. The therapeutic agent for use according to any one of claims 1 to 7, wherein the monoclonal antibody is a chimera.

9. The therapeutic agent for use according to any one of claims 1 to 8, wherein the monoclonal antibody is produced by hybridoma Mouse-Mouse hybridoma deposited under the deposition number NITE BP-972.

10. The therapeutic agent for use according to any one of claims 1 to 9, wherein the antigen binding fragment is Fab, Fab', (Fab')₂, Fv or scFv.

## Patentansprüche

1. Therapeutisches Agens zur Verwendung bei einer Behandlung einer entzündlichen Krankheit, wobei das therapeutische Agens einen monoklonalen Antikörper oder ein Antigen-bindendes Fragment davon umfasst, welcher/welches an ein Peptid bindet, das eine Aminosäuresequenz repräsentiert durch SSVLYGGPPSAA (SEQ ID NR:1) oder ein Konjugat des Peptids umfasst und einen pharmazeutisch annehmbaren Träger,
wobei der monoklonale Antikörper oder das Antigen-bindende Fragment davon eine variable Region der leichten Kette umfasst, welche CDR1 bestehend aus der Aminosäuresequenz repräsentiert durch RASSSVSYMH (SEQ ID NR:2), CDR2 bestehend aus der Aminosäuresequenz repräsentiert durch ATSNLAS (SEQ ID NR:3) und CDR3 bestehend aus einer Aminosäuresequenz repräsentiert durch QQWSSNPWT (SEQ ID NR:4) und eine variable Region der schweren Kette umfassend CDR1 bestehend aus einer Aminosäuresequenz repräsentiert durch GYNMN (SEQ ID NR:7), CDR2 bestehend aus einer Aminosäuresequenz repräsentiert durch NINPYYGSTSYNQKFKG (SEQ ID NR:8) und CDR3 bestehend aus einer Aminosäuresequenz repräsentiert durch SPYYSNYWRYFDY (SEQ ID NR:9) aufweist,
wobei die variable Region der leichten Kette des monoklonalen Antikörpers oder des Antigen-bindenden Fragments davon eine Aminosäuresequenz umfasst, welche durch Position 23 bis Position 128 der SEQ ID NR:6 repräsentiert wird, und
wobei die variable Region der schweren Kette des monoklonalen Antikörpers oder des Antigen-bindenden Fragments davon eine Aminosäuresequenz umfasst, welche durch Position 20 bis Position 141 der SEQ ID NR:11 repräsentiert wird.

2. Therapeutisches Agens zur Verwendung gemäß Anspruch 1, wobei Histon in die entzündliche Krankheit involviert ist.

3. Therapeutisches Agens zur Verwendung gemäß Anspruch 1 oder 2, wobei die entzündliche Krankheit eine akute entzündliche Krankheit ist.

4. Therapeutisches Agens zur Verwendung gemäß einem der Ansprüche 1 bis 3, wobei die entzündliche Krankheit ausgewählt ist aus Sepsis, renalem Ischämie-Reperfusionsschaden und renalem Versagen.

5. Therapeutisches Agens zur Verwendung gemäß einem der Ansprüche 1 bis 4, wobei die entzündliche Krankheit Sepsis ist.

6. Therapeutisches Agens zur Verwendung gemäß einem der Ansprüche 1 bis 5, wobei der pharmazeutisch annehmbare Träger Schlitzschnecken-Hämocyanin, Ovalbumin oder bovines Serumalbumin ist.

7. Therapeutisches Agens zur Verwendung gemäß einem der Ansprüche 1 bis 6, wobei der monoklonale Antikörper oder das Antigen-bindende Fragment davon eine ATP-Synthase-Aktivität herunterregulieren kann.

8. Therapeutisches Agens zur Verwendung gemäß einem der Ansprüche 1 bis 7, wobei der monoklonale Antikörper eine Chimäre ist.

9. Therapeutisches Agens zur Verwendung gemäß einem der Ansprüche 1 bis 8, wobei der monoklonale Antikörper durch Hybridom Maus-Maus-Hybridom, welches unter der Hinterlegungsnummer NITE BP-972 hinterlegt wurde, produziert wird.

10. Therapeutisches Agens zur Verwendung gemäß einem der Ansprüche 1 bis 9, wobei das Antigen-bindende Fragment Fab, Fab', (Fab')₂, Fv oder scFv ist.

## Revendications

1. Agent thérapeutique pour utilisation dans un traitement d'une maladie inflammatoire, qui comprend un anticorps monoclonal ou l'un de ses fragments de liaison à un antigène qui se lie à un peptide comprenant une séquence d'acides aminés représentée par SSVLYGGPPSAA (SEQ ID NO : 1) ou un conjugué du peptide et d'un support pharmaceutiquement acceptable,
dans lequel l'anticorps monoclonal ou l'un de ses fragments de liaison à un antigène comprend une région variable de chaîne légère comprenant une CDR1 constituée d'une séquence d'acides aminés représentée par RASSSVSYMH (SEQ ID NO : 2), une CDR2 constituée d'une séquence d'acides aminés représentée par ATSNLAS (SEQ ID NO : 3), et une CDR3 constituée d'une séquence d'acides aminés représentée par QQWSSNPWT (SEQ ID NO : 4), et une région variable de chaîne lourde comprenant une CDR1 constituée d'une séquence d'acides aminés représentée par GYNMN (SEQ ID NO : 7), une CDR2 constituée d'une séquence d'acides aminés représentée par NINPYYGSTSYNQKFKG (SEQ ID NO : 8), et une CDR3 constituée d'une séquence d'acides aminés représentée par SPYYSNYWRYFDY (SEQ ID NO : 9),
dans lequel la région variable de chaîne légère de l'anticorps monoclonal ou de l'un de ses fragments de liaison à un antigène comprend une séquence d'acides aminés représentée par la position 23 à la position 128 de SEQ ID NO : 6, et
dans lequel la région variable de chaîne lourde de l'anticorps monoclonal ou de l'un de ses fragments de liaison à un antigène comprend une séquence d'acides aminés représentée par la position 20 à la position 141 de SEQ ID NO : 11.

2. Agent thérapeutique pour utilisation selon la revendication 1, dans lequel une histone est impliquée dans la maladie inflammatoire.

3. Agent thérapeutique pour utilisation selon la revendication 1 ou 2, dans lequel la maladie inflammatoire est une maladie inflammatoire aiguë.

4. Agent thérapeutique pour utilisation selon l'une quelconque des revendications 1 à 3, dans lequel la maladie inflammatoire est choisie parmi la septicémie, la lésion rénale d'ischémie-reperfusion et l'insuffisance rénale.

5. Agent thérapeutique pour utilisation selon l'une quelconque des revendications 1 à 4, dans lequel la maladie inflammatoire est la septicémie.

6. Agent thérapeutique pour utilisation selon l'une quelconque des revendications 1 à 5, dans lequel le support pharmaceutiquement acceptable est l'hémocyanine de patelle, l'ovalbumine ou la sérum albumine bovine.

7. Agent thérapeutique pour utilisation selon l'une quelconque des revendications 1 à 6, dans lequel l'anticorps monoclonal ou l'un de ses fragments de liaison à un antigène peut réguler négativement une activité ATP synthase.

8. Agent thérapeutique pour utilisation selon l'une quelconque des revendications 1 à 7, dans lequel l'anticorps monoclonal est une chimère.

9. Agent thérapeutique pour utilisation selon l'une quelconque des revendications 1 à 8, dans lequel l'anticorps monoclonal est produit par un hybridome souris-souris déposé sous le numéro de dépôt NITE BP-972.

10. Agent thérapeutique pour utilisation selon l'une quelconque des revendications 1 à 9, dans lequel le fragment de liaison à un antigène est Fab, Fab', (Fab')₂, Fv ou scFv.
